# EUROPÄISCHE PATENTANMELDUNG

(11) **EP 3 797 677 A1**
(43) Veröffentlichungstag der Anmeldung: **31.03.2021**
(21) Anmeldenummer: 20167087.4
(22) Anmeldetag: 31.03.2020
(51) Int. Cl.: A61B 5/00, A61B 6/00, G05D 1/02

(54) **MOBILES MEDIZINISCHES GERÄT SOWIE VERFAHREN UND SYSTEM ZUR STEUERUNG DES MOBILEN MEDIZINISCHEN GERÄTS**

(30) Priorität: 30.09.2019 EP 19200563
(71) Anmelder: Siemens Healthcare GmbH, 91052 Erlangen (DE)
(72) Erfinder: Garcia, Elmar, 90427 Nürnberg (DE)

(57) **Zusammenfassung**

Die Erfindung betrifft ein Verfahren zur Steuerung eines mobilen medizinischen Geräts (40) umfassend
- eine Sensoreinheit mit zumindest einem Sensor (47) und
- eine Steuerungseinheit (42),
wobei das Verfahren folgende Schritte umfasst:
- Erfassen mittels des zumindest einen Sensors (47) von ersten Sensorsignalen (SES) betreffend wenigstens einen in einer Umgebung des mobilen medizinischen Geräts befindlichen Markers (50), und
Bestimmen mittels der Steuerungseinheit (45) wenigstens eines Steuersignals (STS) für das mobile, medizinische Gerät basierend auf den erfassten ersten Sensorsignalen.

## Beschreibung

Die Erfindung betrifft ein medizinisches Gerät, sowie ein Verfahren und ein System zur Steuerung des mobilen medizinischen Geräts.

Im medizinischen Umfeld werden neben stationären Geräten zunehmend auch mobile medizinische Geräte eingesetzt. Diese mobilen medizinischen Geräte sind typischerweise bewegbar und/oder fahrbar ausgebildet. Solche mobilen medizinischen Geräte können nach Bedarf an verschiedenen Orten eingesetzt werden oder bei Nichtgebrauch temporär aus ihrem Arbeitsumfeld entfernt werden und an einem geeigneten Ort geparkt werden.

Beispielsweise werden für den Transport von Patienten im Klinikbetrieb, möglicherweise zu einer Untersuchung mittels eines medizinischen Bildgebungsgeräts, mobile Patientenlagerungsvorrichtungen eingesetzt. Die Patientenliegen der medizinischen Bildgebungsgeräte selbst können mobil ausgebildet sein, wodurch der Arbeitsablauf vereinfacht werden kann. Weiterhin können medizinische Bildgebungsgeräte mobil ausgebildet sein, wobei insbesondere eine Bildgebungsmodul, bspw. ein Röntgen-C-Bogen oder eine Gantry des medizinischen Bildgebungsgeräts mobil ausgebildet ist. Weiterhin sind mobile Röntgensysteme, mobile Befundungsstationen, mobile Geräte für die Intensivmedizin oder mobile Robotersysteme für medizinische Anwendungen bekannt.

Der Erfindung liegt die Aufgabe zu Grunde, eine verbesserte Steuerung eines medizinischen Geräts anzugeben. Insbesondere liegt die Aufgabe zugrunde, eine Bestimmung von definierten Arbeitspositionen, von Bewegungsbahnen und/oder Gerätekonfigurationen für insbesondere einen (teil-)autonomen Betrieb des mobilen medizinischen Geräts zu vereinfachen.

Die Aufgabe wird durch die Merkmale der unabhängigen Ansprüche gelöst. Vorteilhafte Ausgestaltungen sind in den Unteransprüchen beschrieben.

Nachstehend wird die erfindungsgemäße Lösung der Aufgabe in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

In einem ersten Aspekt betrifft die vorliegende Erfindung ein Verfahren zur Steuerung eines mobilen medizinischen Geräts.

Das mobile medizinische Gerät kann grundsätzlich als Patientenliege, Patientenlagerungstisch, Bildgebungsanlage, medizinische Robotervorrichtung oder Assistenzanlage für eine medizinische Bildgebung oder eine medizinische Behandlung inklusive eines medizinischen Eingriffs ausgebildet sein. Das mobile medizinische Gerät dient einem medizinischen Zweck. Das Gerät ist dadurch gekennzeichnet, dass es mobil ausgebildet ist. Eine mobile Ausgestaltung umfasst bevorzugt, dass sich da mobile Gerät in seiner Gesamtheit in bzw. auf einer Fahrebene in wenigstens eine Raumrichtung in der Ebene bewegen oder in dieser Ebene drehen kann. Eine mobile Ausgestaltung umfasst grundsätzlich auch eine Bewegung des Geräts in seiner Gesamtheit in einer dritten Raumrichtung, also außerhalb der Fahrebene. Die mobile Ausgestaltung umfasst alternativ oder zusätzlich auch eine Bewegung einzelner Gerätekomponenten in allen drei Raumrichtungen. Bspw. kann eine Bewegung lediglich eines Roboterarms, einer Strahler-Detektor-Einheit einer Röntgen-Bildgebungsanlage oder einer Patientenauflagefläche einer Patientenliege von der mobilen Ausgestaltung umfasst sein.

Die mobile Ausgestaltung des medizinischen Geräts kann eine aktive, automatische, mit anderen Worten autonome Bewegung des Geräts und/oder eine passive, geführte bzw. durch einen Benutzer geführte bzw. initiierte Bewegung umfassen. Insbesondere ist eine automatische, jedoch durch einen Benutzer, bspw. durch Berührung des medizinischen Geräts, autorisierte Bewegung umfasst.

Das erfindungsgemäße mobile medizinische Gerät umfasst eine Sensoreinheit, diese kann einen oder mehrere Sensoren umfassen. Der Sensor ist ausgebildet, zumindest erste Sensorsignale in Bezug auf wenigstens einen in der Umgebung des mobilen Geräts befindlichen Marker zu erfassen. Der Sensor ist also eingerichtet, den wenigstens einen Marker in seiner Umgebung zu detektieren bzw. zu erkennen.

Der erfindungsgemäße Marker ist so ausgebildet, dass er wenigstens eine Positions-, Ausrichtungs-, oder Orientierungsmarkierung des Markers umfasst, die mittels wenigstens eines Sensors der Sensoreinheit detektiert werden kann. Der Marker befindet sich der Umgebung, insbesondere in (unmittelbarer) räumlicher Nähe zu dem mobilen Gerät, bzw. der Sensoreinheit. Der Marker ist in bevorzugten Ausführungen in Bezug auf seinen direkten Untergrund, auf dem er angeordnet ist ortsfest ausgebildet, das heißt er ist in Bezug auf den Untergrund nicht beweglich. Bevorzugt ist die Markerposition so gewählt, dass der Sensor den Marker bei einer Standard-Bewegung des mobilen medizinischen Geräts zwangsläufig detektiert. Bspw. kann der Marker am Boden eines Behandlungs- oder Untersuchungsraumes in einer medizinischen Einrichtung angeordnet sein, über welchen sich das medizinische Gerät zwangläufig bewegt, wenn es in den Raum einfährt. In Ausführungen ist der Marker als flächiger Marker ausgebildet, sprich, er weist eine Ausdehnung in zwei Raumrichtungen auf. In Ausführungen der Erfindung ist lediglich einer, in anderen Ausführungen sind mehrere Marker, insbesondere zwei oder drei Marker vorgesehen.

Der erfindungsgemäße Marker ist insbesondere ausgebildet, in einer medizinischen Umgebung eingesetzt zu werden. Der Marker kann also insbesondere ausgebildet sein, in einer medizinischen Einrichtung, bspw. einem Krankenhaus oder einem Radiologie-Zentrum angebracht zu werden. Er ist insbesondere so ausgebildet, dass er eine Bewegung des medizinischen Geräts nicht behindert oder stört.

In anderen Ausführungen der Erfindung ist der wenigstens eine Marker in einem Boden, an einer Wand oder einer Decke eines Untersuchungsraumes einer medizinischen Einrichtung angeordnet. Der Marker kann auf einen Untergrund aufgeklebt, eingraviert, aufgezeichnet oder dergleichen werden. Die Ausgestaltung bzw. die Aufbringungsart des Markers kann insbesondere von der Beschaffenheit des Untergrunds abhängen. In anderen Ausführungen der Erfindung kann wenigstens ein Marker an einem zweiten mobilen oder einem stationären Gerät in der Umgebung des mobilen Geräts angeordnet sein.

Der einzelne Sensor der Sensoreinheit kann als optischer, insbesondere als zweidimensionaler oder dreidimensionaler optischer Sensor, Lasermesssensor, akustischer Sensor, Magnetfeldsensor, elektrischer Feldsensor, Induktionssensor, Funkwellensensor oder dergleichen ausgebildet sein. Der Sensor kann als berührungsloser, beispielsweise optischer, oder als taktiler Sensor ausgebildet sein. Die Sensoreinheit kann mehrere gleiche oder verschiedene einzelne Sensoren umfassen.

Das erfindungsgemäße Verfahren zur Steuerung eines mobilen medizinischen Geräts umfasst folgende Schritte:
- Erfassen mittels des zumindest einen Sensors von ersten Sensorsignalen betreffend wenigstens einen in einer Umgebung des mobilen medizinischen Geräts befindlichen Markers, und
- Bestimmen mittels der Steuerungseinheit wenigstens eines Steuersignals für das mobile, medizinische Gerät basierend auf den erfassten ersten Sensorsignalen.

Die ersten Sensorsignale umfassen wenigstens eine Information bezüglich der Position, der Orientierung und/oder der Ausrichtung des Markers. Die erfassten, ersten Sensorsignale werden über eine Schnittstelle an eine erfindungsgemäße Steuerungseinheit übertragen. Dies Steuerungseinheit bestimmt unter Auswertung der ersten Sensorsignale wenigstens ein, bevorzugt mehrere Steuersignale für das mobile Gerät. Die Steuersignale betreffen eine Bewegung des mobilen Geräts. Die Steuersignale können in Ausführungen auch einen Betrieb, einen Betriebszustand und/oder eine Konfiguration des mobilen Geräts betreffen.

Die Steuersignale werden erfindungsgemäß an das mobile Gerät ausgegeben. Mit anderen Worten wird eine Bewegung des mobilen medizinischen Geräts mittels des Steuersignals gesteuert.

In einem weiteren Aspekt betrifft die vorliegende Erfindung das oben beschriebene mobile medizinische Gerät. Das mobile medizinische Gerät umfasst
- eine Sensoreinheit mit dem zumindest einen Sensor zum Erfassen wenigstens erster Sensorsignale, und
- eine erfindungsgemäße Steuerungseinheit zur Steuerung des mobilen medizinischen Geräts.

Die Steuerungseinheit ist ausgebildet, Schritte des erfindungsgemäßen Verfahrens durchzuführen. Die Steuerungseinheit umfasst zu diesem Zweck Mittel zum Ausführen von Schritten des erfindungsgemäßen Verfahrens. Die Steuerungseinheit umfasst
- eine Schnittstelle zum Empfangen von mittels des zumindest einen Sensors erfassten ersten Sensorsignalen betreffend wenigstens einen in einer Umgebung des mobilen medizinischen Geräts befindlichen Markers,
- eine Bestimmungseinheit zum Bestimmen von wenigstens einem Steuersignal für das mobile, medizinische Gerät basierend auf den erfassten ersten Sensorsignalen, und
- eine Schnittstelle zum Ausgeben des wenigstens einen Steuersignals an das mobile Gerät.

Vorteilhaft ist die Steuerungseinheit in das mobile medizinische Gerät integriert. Alternativ kann die Steuerungseinheit auch entfernt bzw. abgelegen von dem mobilen Gerät angeordnet sein. Die Steuerungseinheit ist insbesondere ausgebildet, unter Auswertung der erfassten ersten Sensorsignale den Schritt des Bestimmens von Steuersignalen für das mobile Gerät durchzuführen.

Die Steuerungseinheit kann insbesondere als oder als Teil einer Recheneinheit des mobilen medizinischen Geräts ausgebildet sein. In einer bevorzugten Ausführung ist die Steuerungseinheit mit dem mobilen medizinischen Gerät verbunden. Besonders vorteilhaft ist die Steuerungseinheit in das mobile medizinische Gerät integriert. Alternativ kann die Steuerungseinheit auch entfernt bzw. abgelegen davon angeordnet sein. Die Steuerungseinheit kann ausgebildet sein, insbesondere den Schritt des Bestimmens von wenigstens einem Steuersignal für das mobile, medizinische Gerät basierend auf den erfassten ersten Sensorsignalen für ein mobiles medizinisches Gerät oder für eine Vielzahl von mobilen medizinischen Geräten, bspw. in Form einer Geräte-Flotte, bspw. in einem Radiologie-Zentrum oder Krankenhaus umfassend mehrere mobile medizinische Geräte, durchzuführen.

In einem weiteren Aspekt betrifft die vorliegende Erfindung ein System zur Steuerung eines mobilen medizinischen Geräts. Dieses umfasst
- ein erfindungsgemäßes mobiles medizinisches Gerät samt erfindungsgemäßer Steuerungseinheit, und
- wenigstens einen in einer Umgebung des mobilen medizinischen Geräts befindlichen Marker, der wie eingangs beschrieben ausgebildet ist.

In Ausführungen der Erfindung kann das System auch mehrere mobile medizinische Geräte umfassen, die allesamt mit demselben, wenigstens einen Marker arbeiten. Alternativ kann für jedes der mobilen medizinischen Geräte ein eigener Marker bzw. eine Vielzahl von Markern vorgesehen sein.

In besonders bevorzugten Ausführungen der Erfindung ist der wenigstens eine Marker als QR-Marker ausgebildet. Dieser ist insbesondere geeignet, Positions- oder Orientierungsinformation bezüglich des Markers zu kodieren. Darüber hinaus sind QR-Marker auch geeignet, weitere Daten für eine sensorische Erfassung zu codieren, die dann mittels Sensor erfasst werden können. Die Verwendung von QR-Markern ermöglicht vorteilhaft eine einfache und robuste Möglichkeit zur Datenspeicherung, Datencodierung und flexiblen Datenübertragung an ein mobiles medizinisches Gerät. Derart können Zusatzinformationen bspw. in Bezug auf die Umgebung des mobilen Geräts, einen medizinischen Ablaufplan (workflow), Assistenzfunktionen oder automatisierte Vorgänge ermöglichen. Automatische Vorgänge bezeichnen Aktionen, insbesondere Bewegungen, die das mobile Gerät autonom, d.h. selbständig und ohne Unterstützung oder Begleitung eines Nutzers ausführt. Assistenzfunktionen bezeichnen Aktionen, insbesondere Bewegungen, die durch das mobile Gerät unter Nutzer-Führung, Nutzer-Aufsicht oder Nutzer-Unterstützung durchgeführt werden.

In alternativen Ausführungen kann der Marker auch als Strich- oder Barcode-Marker oder in Form von DataMatrix-Code ausgebildet sein. Insbesondere ein QR-Code und ein DataMatrix-Code sind besonders gut für die Ausbildung eines erfindungsgemäßen Markers ausgebildet, da sie über sehr robuste Fehlerkorrektur-Vorschriften verfügen.

Die Erfindung realisiert über die Verwendung und das Auslesen des wenigstens einen Markers eine einfach zu handhabende Orientierung des mobilen Geräts, insbesondere eine autonome Orientierung des mobilen Geräts ohne eine manuelle Führung. Insbesondere ermöglicht die Erfindung eine zuverlässige und wiederholbare Orientierung und Bewegung des mobilen Geräts in einer Krankenhausumgebung.

In Ausführungen der vorliegenden Erfindungen umfasst das mobile medizinische Gerät ein Fahrwerk und/oder eine mobile Komponente und die Steuerungseinheit ist ausgebildet, basierend auf den bestimmten Steuersignalen das Fahrwerk und/oder die mobile Komponente des mobilen Geräts zu steuern.

Das Fahrwerk kann zumindest ein Fahrmittel, beispielsweise zumindest eine möglichst leichtgängige Rolle und/oder zumindest ein Rad, umfassen. Die Bewegung des mobilen medizinischen Geräts kann autonom oder zumindest teilweise von einem Bedienpersonal gesteuert werden, wobei das Fahrwerk motorisch unterstützt ausgebildet sein kann. Das Fahrwerk ist vorteilhafterweise als omnidirektionales Fahrwerk ausgebildet. Das heißt, dass das Fahrwerk eine Bewegung des mobilen Geräts in jeder beliebigen Richtung auf der Fahrebene ermöglichen kann. Insbesondere ist dabei für eine Richtungsänderung der Bewegung des medizinischen Geräts keine Drehung des medizinischen Geräts erforderlich. Die Richtungsänderung des medizinischen Geräts kann somit direkt erfolgen. Das Fahrwerk, insbesondere die Fahrmittel des Fahrwerks, kann also seine Orientierung auf der Fahrebene in jede beliebige Richtung verändern. Gleichzeitig ist derart möglich, dass die Position der Drehachse bezüglich des medizinischen Geräts beliebig festgelegt werden kann. Die Drehachse bewegt sich dabei typischerweise mit dem medizinischen Gerät mit. Die Drehachse behält vorzugsweise ohne Einwirkung von außen ihre Position bezüglich des medizinischen Geräts bei. Die Fahrebene ist dabei insbesondere anhand des Bodens, auf welchem das medizinische Gerät bewegt wird, vorgegeben.

Eine erfindungsgemäße mobile Komponente ist als ein Bestandteil oder Bauteil bzw. Gruppe aus mehreren Bestandteilen oder Bauteilen des mobilen Geräts ausgebildet, wobei die mobile Komponente individuell relativ zum Rest des mobilen Geräts bewegbar oder verfahrbar ist. Bspw. ist die mobile Komponente als mobile Patientenliege ausgebildet, die gegenüber einem verfahrbaren Patiententisch (mobiles Gerät) verstellbar ist. Die mobile Komponente kann auch als ein Roboterarm einer mobilen Assistenz-Vorrichtung ausgebildet sein, wobei der Roboterarm bspw. ausgebildet ist, während eines medizinischen Eingriffs medizinischem Personal zu assistieren, bspw. medizinische Instrumente zu halten. Alternativ kann die mobile Komponente auch als Gantry bzw. C-Bogen umfassend wenigstens ein Röntgen-Strahler-Detektor-Paar einer mobilen Röntgen- bzw. Computertomographie-Anlage ausgebildet sein, wobei sich Gantry oder C-Bogen im Verlauf einer medizinischen Bildgebung Entsprechend einer Bildgebungstrajektorie verstellen.

Die Steuerungseinheit ist insbesondere derart auf das Fahrwerk des mobilen Geräts abgestimmt, dass es die Steuerung des Fahrwerks, insbesondere des omnidirektionalen Fahrwerks, ermöglicht. Dafür kann die Steuerungseinheit gleichzeitig zumindest zwei, vorzugsweise alle Rollen des Fahrwerks, beispielsweise für eine Drehung des medizinischen Geräts auf der Fahrebene, ansteuern. Somit ist die Steuerungseinheit vorteilhafterweise derart auf das Fahrwerk abgestimmt, dass die Steuerungseinheit besonders einfach die Bewegung des medizinischen Geräts in zumindest zwei Raumrichtungen auf der Fahrebene und/oder eine Drehbewegung des medizinischen Geräts ermöglicht. Die Steuerungseinheit kann automatisch ausgebildet sein. Vorteilhafterweise ist die Steuerungseinheit gleichzeitig automatisch und manuell ausgebildet, so dass eine manuelle Steuerung des Fahrwerks mittels der Steuerungseinheit einer automatischen Steuerung des Fahrwerks überlagert werden kann. Die Steuerungseinheit ist insbesondere dazu ausgebildet Steuerungssignale an das Fahrwerk, möglicherweise an einen das Fahrwerk antreibenden Motor, zu senden. Die Steuerungseinheit kann weiterhin vorteilhafterweise dazu ausgebildet sein, Steuerungssignale von einem Bedienpersonal, insbesondere über eine Schnittstelle, bspw. in Form von Bedienelementen wie Bedienknöpfen, Griffen oder eines graphischen Bedienfeldes zu erhalten.

Die Steuerungseinheit ist ferner insbesondere derart auf die mobile Komponente des mobilen Geräts abgestimmt, dass es die Steuerung der mobilen Komponente ermöglicht. Somit ist die Steuerungseinheit vorteilhafterweise derart auf die mobile Komponente abgestimmt, dass die Steuerungseinheit besonders einfach die Bewegung der mobilen Komponente relativ zum mobilen Gerät in zumindest zwei, bevorzugt drei Raumrichtungen, oder eine Drehbewegung der mobilen Komponente ermöglicht. Auch in Bezug auf die mobile Komponente kann die Steuerungseinheit automatisch ausgebildet sein. Vorteilhafterweise ist die Steuerungseinheit gleichzeitig automatisch und manuell ausgebildet, so dass eine manuelle Steuerung der mobilen Komponente mittels der Steuerungseinheit einer automatischen Steuerung der mobilen Komponente überlagert werden kann. Die Steuerungseinheit ist insbesondere dazu ausgebildet Steuerungssignale an die mobile Komponente, möglicherweise an einen die Komponente antreibenden Motor, zu senden. Die Steuerungseinheit kann weiterhin vorteilhafterweise dazu ausgebildet sein, Steuerungssignale von einem Bedienpersonal, insbesondere über eine Schnittstelle, zu erhalten.

In Ausführungen des erfindungsgemäßen Verfahrens umfasst das Bestimmen des Steuersignals, basierend auf den ersten Sensorsignalen wenigstens eine Position und/oder eine Orientierung für das mobile Gerät relativ zu der Position des Markers zu bestimmen. Die ersten Sensorsignale umfassen vorteilhaft Positionsdaten für das mobile Gerät. Die ersten Sensorsignale definieren vorteilhaft auch eine Ebene, insbesondere eine Fahrebene für das mobile Gerät. In bevorzugten Ausführungen definiert die Position des Markers eine Startposition für das mobile Gerät, bspw. eine Startposition für eine medizinische Behandlung oder Untersuchung. Die Startposition kann der Position des Markers entsprechen oder eine relativ zu der Markerposition in der definierten Ebene verschobene Position des mobilen medizinischen Geräts sein. Alternativ kann die Position des Markers eine Stopp-Position für das mobile Gerät bestimmen. Aus der Positionsinformation bezüglich des Markers und einer bspw. mittels Steuerungseinheit überwachten Position des mobilen Geräts kann die Steuerungseinheit bspw. eine Bahn bzw. Trajektorie ermitteln, die das mobile Gerät zurücklegen muss, um die Startposition einzunehmen und ein entsprechendes Steuersignal für das mobile Gerät, insbesondere sein Fahrwerk ermitteln. Alternativ oder zusätzlich umfassen die ersten Sensorsignale auch Informationen in Bezug auf eine Orientierung des mobilen Geräts. Eine Orientierung kann eine bestimmte Ausrichtung des mobilen Geräts in der Fahrebene sein, die bspw. mittels Drehbewegung des mobilen Geräts in der Fahrebene eingenommen werden kann. Die Orientierung kann auch eine vordefinierte Stellung des mobilen Geräts und/oder seiner mobilen Komponente umfassen, die bspw. in Vorbereitung oder nach Beendigung einer medizinischen Untersuchung in eine Ausgangsstellung verbracht werden muss.

In weiteren Ausführungen des erfindungsgemäßen Verfahrens umfasst das Bestimmen des Steuersignals, basierend auf den ersten Sensorsignalen wenigstens ein Referenz-Koordinatensystem für das mobile Gerät zu definieren. Bei dem Referenz-Koordinatensystem handelt es sich um ein drei-dimensionales, globales Koordinatensystem. In diesen Ausführungen sind wenigstens zwei jeweils zwei-dimensionale Marker oder drei eindimensionale Marker vorgesehen, die relativ zu einander so angeordnet sind, dass sie jeweils gemeinsam die drei Achsen des Referenz-Koordinatensystems definieren. Die Definition eines drei-dimensionalen Referenz-Koordinatensystems ermöglicht vorteilhaft eine Bewegung des mobilen Geräts und/oder einer mobilen Komponente in allen drei Raumrichtungen.

In Ausführungen des erfindungsgemäßen Verfahrens umfasst das Bestimmen des Steuersignals ferner, basierend auf den ersten Sensorsignalen eine Arbeitsposition und/oder einen Bewegungspfad des mobilen Geräts zu ermitteln, insbesondere kann eine Arbeitsposition und/oder ein Bewegungspfad für eine mobile Komponente des mobilen Geräts ermittelt werden. Insbesondere können basierend auf den ersten Sensorsignalen eine Vielzahl von Arbeitspositionen ermittelt werden, die zusammen betrachtet einen Bewegungspfad für die mobile Komponente definieren.

Mit anderen Worten definieren die Sensorsignale vorteilhaft, wohin eine mobile Komponente verstellt werden soll oder entlang welcher Trajektorie diese bewegt oder verfahren werden soll. Arbeitspositionen einer mobilen Komponente können insbesondere Positionen einer relativ zum Patiententisch verschieblichen Patientenliege sein, oder Stellwinkel eines C-Bogens eines mobilen Röntgengeräts betreffen. Insbesondere können die Arbeitspositionen und/oder der Bewegungspfad in Bezug auf das globale Referenz-Koordinatensystem in drei Raumdimensionen einstellbar sein.

In besonders bevorzugten Ausführungen der vorliegenden Erfindung umfasst das Bestimmen des Steuersignals ein Bestimmen einer Position und/oder Orientierung des mobilen Geräts und ein Bestimmen einer Arbeitsposition und/oder eines Bewegungspfads einer mobilen Komponente.

In anderen Ausführungen des erfindungsgemäßen Verfahrens wird eine zuvor bestimmte Position, Orientierung, Arbeitsposition und/oder ein zuvor bestimmter Arbeitspfad basierend auf erfassten zweiten Sensorsignalen betreffend wenigstens einen in der Umgebung des mobilen Geräts befindlichen zweiten Marker verändert. Es kann folglich mindestens ein zweiter Marker vorgesehen sein, der ebenfalls mittels des wenigstens eines Sensors detektiert werden kann, was eine Erfassung von zweiten Sensorsignalen durch den Sensor bewirkt. Der zweite Marker wird vorteilhaft nach Detektion des ersten Markers detektiert. Bspw. kann der zweite Marker entlang einer ermittelten Bahn für das mobile Gerät hin zu einer bestimmten Position für das mobile Gerät detektiert werden. Der zweite Marker kann bevorzugt auf oder an einem weiteren mobilen Gerät angeordnet sein. Die bereits basierend auf den ersten Sensorsignalen bestimmten Steuersignale können nun bspw. im Sinne einer Kollisionsüberwachung verändert werden, sodass sich bspw. das mobile Gerät entlang einer veränderten Bahn zur bestimmten Position bewegt und dadurch das zweite mobile Gerät umfährt.

In Ausführungen des erfindungsgemäßen Verfahrens umfasst das Bestimmen des Steuersignals, basierend auf den Sensorsignalen wenigstens einen Konfigurationsparameter für das mobile Gerät zu bestimmen. Vorteil des erfindungsgemäßen, wenigstens einen Markers ist die Möglichkeit, weitere Zusatzinformation für das mobile Gerät zu codieren und dem mobilen Gerät über die Sensorsignale zur Verfügung zu stellen. Die Sensorsignale können in dieser Ausführung einen Konfigurationsparameter für das mobile Gerät angeben. Ein erfindungsgemäßer Konfigurationsparameter ist dazu ausgebildet, eine (Vor-)Konfiguration eines mobilen Gerätes vorzunehmen. Mit anderen Worten kann das mobile Gerät basierend auf einem Konfigurationsparameter konfiguriert, voreingestellt bzw. in einen bestimmten Betriebsmodus versetzt werden. Der Konfigurationsparameter ist bspw. kennzeichnend für einen bestimmten Untersuchungs- oder Behandlungsraum, in welchem der Marker und das mobile Gerät angeordnet sind. Der Konfigurationsparameter kann alternativ bspw. kennzeichnend sein für eine Untersuchungs- oder Behandlungsart, die mit dem mobilen Gerät durchgeführt werden soll oder kennzeichnend für einen Nutzer sein, der eine Untersuchung oder Behandlung durchführt. Über die Codierung von Zusatzinformation in dem wenigstens einen Marker kann eine manuelle Konfiguration des mobilen Geräts entfallen, was Zeit spart und die Bedienerfreundlichkeit erhöht.

In bevorzugten Ausführungen des erfindungsgemäßen Verfahrens legt der Konfigurationsparameter eine Konfiguration des mobilen Geräts bezüglich einer Netzwerkanbindung fest. Der erfindungsgemäße Marker codiert in dieser Ausführungsvariante bspw. an den Untersuchungsraum gekoppelte Zusatzinformationen bezgl. der dort bestehenden Netzwerk-Verbindung. Die erfassten Sensorsignale umfassen folglich Angaben zu einem in dem Untersuchungsraum bestehenden Netzwerk und können bspw. auch Daten für eine Zugriffsberechtigung auf das Netzwerk für das mobile Gerät umfassen, bspw. einen Netzwerkschlüssel und Autorisierungsdaten, um sich dem Netzwerk gegenüber zu identifizieren und/oder zu autorisieren. Derart kann sich das mobile Gerät nach Detektion des ersten Markers direkt und automatisch für einen weiteren Datenaustausch in das Netzwerk einwählen. Insbesondere handelt es sich bei dem Netzwerk um ein kabelloses Internet oder Intranet, bspw. kann es als wireless LAN (WLAN oder WiFi) ausgebildet sein. Das Netzwerk kann eine Kombination aus verschiedenen Netzwerkbeispielen umfassen.

In Ausführungen des erfindungsgemäßen Verfahrens legt der Konfigurationsparameter eine Konfiguration des mobilen Geräts bezüglich eines Nutzers fest. Insbesondere legt der Konfigurationsparameter in dieser Ausführung einen Benutzerabhängigen Betriebsmodus fest. Die Benutzerabhängigkeit definiert bevorzugt von einem bestimmten Nutzer präferierte Geräteeinstellungen. Bspw. können die ersten Sensorsignale Identifikationsdaten eines Nutzers umfassen und die Steuerungseinheit kann ausgebildet sein, basierend auf den erfassten Identifikationsdaten ein Steuersignal für das mobile Gerät zu ermitteln, welches die präferierten Geräteeinstellung dieses Nutzers berücksichtigt. So kann bspw. eine motorische Bewegungsunterstützung des mobilen Geräts in Abhängigkeit des identifizierten Nutzers verstärkt oder vermindert werden, sodass der Nutzer beim (teil-)autonomen Bewegen des mobilen Geräts oder einer mobilen Komponente, bspw. der Patientenliege, mehr oder weniger Kraft aufwenden oder die Bewegung der mobilen Komponenten lediglich mittels Berührung zur Autorisierung begleiten muss. Damit wird vorteilhaft die Bedienerfreundlichkeit erhöht.

In Ausführungen des erfindungsgemäßen Verfahrens legt der Konfigurationsparameter eine Konfiguration des mobilen Geräts bezüglich eines medizinischen Workflows fest. Ein medizinischer Workflow, also ein medizinischer Untersuchungs- oder Behandlungsablauf kann bspw. eine Kommunikation bzw. einen Datenaustausch mit anderen mobilen oder stationären Geräten in dem Untersuchungsraum erfordern. Alternativ oder zusätzlich können in Abhängigkeit des Workflows weitere, jedoch manuell durchzuführende Vorbereitungshandlungen erforderlich sein. Über mittels der Sensorsignale codierte Zusatzinformation zu dem medizinischen Workflow kann die Steuerungseinheit vorteilhaft Steuersignale bestimmen, die automatisch den Aufbau einer Datenverbindung zwischen dem mobilen Gerät und dem weiteren in den Workflow involvierten Gerät bewirken oder bspw. ein akustisches oder optisches Warnsignal an einen Nutzer ausgeben, die auf die weiteren notwendigen Vorbereitungshandlungen hinweisen.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine Ansicht eines erfindungsgemäßen Systems umfassend ein erfindungsgemäßes mobiles Gerät in Form eine C-Bogen-Röntgengeräts gemäß einer Ausführungsform der vorliegenden Erfindung, und
- FIG 2: eine Ansicht eines erfindungsgemäßen Verfahrens gemäß einem Ausführungsbeispiel der vorliegenden Erfindung.

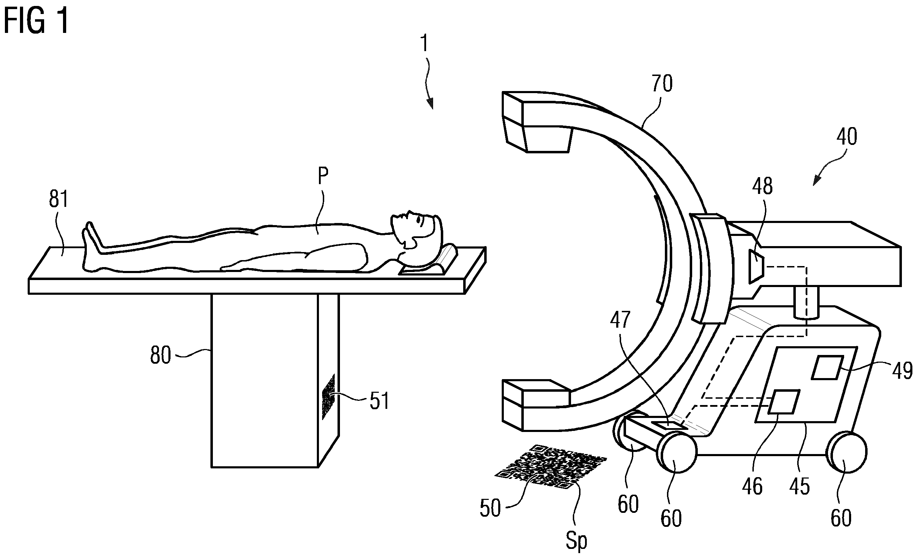

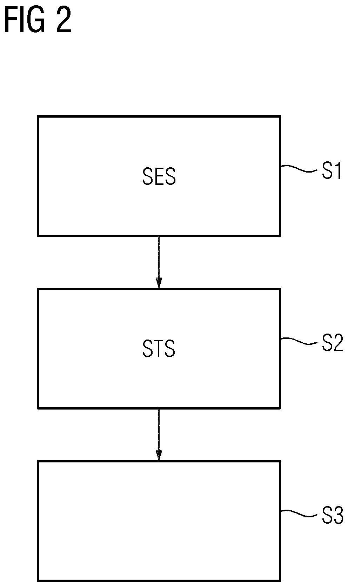

**Figur 1** zeigt eine Ansicht eines erfindungsgemäßen Systems 1 umfassend ein erfindungsgemäßes mobiles Gerät (40) in Form eine C-Bogen-Röntgengeräts gemäß einer Ausführungsform der vorliegenden Erfindung. Das mobile Gerät umfasst eine mobile Komponente 70 in Form eines Röntgen-C-Bogens, der an seinen Bogenenden einen Röntgenstrahler und einen gegenüberliegenden Röntgendetektor umfasst. Der Röntgen-C-Bogen 70 ist relativ zum mobilen Gerät verfahrbar bzw. verstellbar. In dieser Ausführung ist der Röntgen-C-Bogen 70 dreh- bzw. verschwenkbar ausgebildet, sodass die Strahler-Detektor-Anordnung in verschiedenen Stellwinkeln relativ zur durch den Boden gebildeten horizontalen Ebene positioniert werden kann, um medizinische Bilddaten eines Patienten P zu erzeugen. Das System umfasst zudem einen am Boden flächig angeordneten Marker 50, der in Form eines QR-Codes ausgebildet ist. Der QR-Marker ist an einer Position angeordnet, die einer Startposition SP des mobilen Geräts 40 entspricht. Der QR-Marker 50 codiert über seine Achsen und seine Ausrichtung am Boden eine Fahrebene für das mobile Gerät, in dieser Ausführung entspricht die Fahrebene der durch den Boden des Untersuchungsraumes gebildeten horizontalen Ebene. Das mobile Gerät umfasst eine Sensoreinheit, die zwei Sensoren 47 und 48 umfasst. Während die Kamera 47 auf den Boden des Untersuchungsraumes ausgerichtet ist, um den Boden optisch zu scannen, scannt Kamera 48 die Umgebung in einer horizontalen Richtung. Sensoren 47 und 48 sind als optische Sensoren in Form von optischen Kameras ausgebildet. Sensoren 47 und 48 sind vorteilhaft immer aktiv, sodass sie ihre Umgebung permanent auf Sensorsignale SES scannen. Sobald sich QR-Marker 50 im Sichtfeld der optischen Kamera 47 befindet, bspw. dann, wenn das mobile Gerät 40 näher an einen in räumlicher Nähe angeordneten Patiententisch 80 durch einen Nutzer ggf. motorisch unterstützt heran geschoben wird, erfasst die Kamera 47 erste Sensorsignale SES in Form von optischen Bilddaten. Diese werden über eine Schnittstelle 46 an eine Steuerungseinheit 45 des mobilen Geräts 40 übertragen. Steuerungseinheit 45 umfasst eine Bestimmungseinheit 49, die ausgebildet ist, basierend auf den Sensorsignalen SES Steuersignale für das mobile Gerät 40, insbesondere für das Fahrwerk 60 oder die mobile Komponente 70 zu bestimmen, wie mit Bezug zu Figur 2 noch näher erläutert wird. Kamera 48 ist insbesondere ausgebildet, einen zweiten Marker 51, ebenfalls als QR-Code ausgebildet zu erkennen, der an einem Patiententisch 80 umfassend eine demgegenüber verschiebbar ausgebildete Patientenliege 81 angeordnet ist. Kamera 48 steht ebenfalls mit der Steuerungseinheit 45 über Schnittstelle 46 in Datenverbindung.

Die Sub-Komponenten 46, 49 der Steuerungseinheit 45 stehen jeweils in Datenaustausch mit einander. Über Schnittstelle 46 steht die Steuerungseinheit 45 mit weiteren Komponenten des mobilen Geräts 40, insbesondere mit dem Fahrwerk 60 und der mobilen Komponente 70 in Verbindung.

Die Schnittstelle 46 kann als Hardware- oder Software-Schnittstelle ausgebildet sein, bspw. als PCI-Bus, USB oder Firewire. Ein Datenaustausch erfolgt bevorzugt mittels einer Netzwerk-Verbindung. Das Netzwerk kann als local area network (LAN), bspw. Ein Intranet oder ein wide area network (WAN) ausgebildet sein. Die Netzwerkverbindung ist bevorzugt kabellos ausgebildet, bspw. als wireless LAN (WLAN oder WiFi). Das Netzwerk kann eine Kombination aus verschiedenen Netzwerkbeispielen umfassen. Datenübertragung kann basierend auf einer Datenabfrage oder eigeninitiativ erfolgen. Datenübertragung zwischen zwei Einheiten bzw. Systemkomponenten kann bidirektional oder unidirektional erfolgen.

Die Steuerungseinheit 45 kann in Form von Hard- oder in Form von Software ausgebildet sein. Beispielsweise ist die Steuerungseinheit 45 als ein sogenanntes FPGA (Akronym für das englischsprachige "Field Programmable Gate Array") ausgebildet oder umfasst eine arithmetische Logikeinheit. Die Steuerungseinheit 45, einzelne oder alle ihrer Sub-Komponenten können alternativ dezentral angeordnet sein, z.B. können einzelne Rechenschritte des Verfahrens in einem zentralen Rechenzentrum einer medizinischen Dienstleistungseinrichtung, z.B. ein Krankenhaus, oder in der Cloud ausgeführt werden. Hierbei sind insbesondere Daten- und Patientenschutz beim Datenaustausch zu berücksichtigen. Die Steuerungseinheit 45 kann auch als Cloud-basierter Computer ausgebildet sein.

**Figur 2** zeigt eine Ansicht eines erfindungsgemäßen Verfahrens gemäß einem Ausführungsbeispiel der vorliegenden Erfindung.

Das Verfahren dient einer Steuerung eines mobilen medizinischen Geräts umfassend
- eine Sensoreinheit mit zumindest einem Sensor 47 und
- eine Steuerungseinheit 42. Das Verfahren umfasst eine Vielzahl von Schritten.

In einem ersten Schritt S1 erfolgt ein Erfassen mittels des zumindest einen Sensors 47 von ersten Sensorsignalen (SES) betreffend wenigstens einen in einer Umgebung des mobilen medizinischen Geräts befindlichen Markers 50. Die ersten Sensorsignale SES umfassen eine Information bezüglich der Position, der Orientierung und/oder der Ausrichtung des Markers 50.
In einem zweiten Schritt erfolgt ein Bestimmen mittels der Steuerungseinheit 45 wenigstens eines Steuersignals STS für das mobile, medizinische Gerät 40 basierend auf den erfassten ersten Sensorsignalen SES.

In einem dritten Schritt S3 erfolgt optional ein Ausgeben des Steuersignals an das mobile Gerät 40, insbesondere an das Fahrwerk 60 und/oder an die mobile Komponente 70. Das Steuersignal STS trägt vorteilhaft Positions-, Orientierungs- und/oder Zusatzinformationen Rechnung, die mittels des Markers 50 kodiert sind und über die Sensorsignale SES der Steuerungseinheit 45 zugänglich gemacht werden.

Das erfindungsgemäße Verfahren kann vorteilhaft weitere Verfahrens-(unter-)schritte umfassen.

Beispielsweise kann Schritt S2 zum Bestimmen des Steuersignals STS umfassen, basierend auf den ersten Sensorsignalen SES wenigstens eine Position und/oder eine Orientierung für das mobile Gerät 40 relativ zu der Position des Markers 50 zu bestimmen. Mit anderen Worten umfasst Schritt S2 ein Bestimmen einer Relativposition des mobilen Geräts 40 zu dem Marker 50. Die Relativposition kann einer Start- bzw. einer Stopp-Position des mobilen Geräts 40 für einen medizinischen Workflow entsprechen. Mit den (ersten) Sensorsignalen SES kann in Schritt S2 auch die Fahrebene für das mobile Gerät 40 definiert werden, die typischerweise durch den Untergrund bzw. den Boden des Untersuchungsraumes vorgeben ist. Umfassen die ersten Sensorsignale SES Orientierungsinformationen des ersten Markers 50, kann Schritt S2 ein Bestimmen einer einzunehmenden Orientierung für das mobile Gerät 40 umfassen. Eine Orientierung kann eine bestimmte Ausrichtung des mobilen Geräts in der Fahrebene sein, die bspw. mittels Drehbewegung des mobilen Geräts in der Fahrebene eingenommen werden kann. Die Orientierung kann auch eine vordefinierte Stellung des mobilen Geräts 40 und/oder seiner mobilen Komponente 70 umfassen.

Schritt S2 kann auch umfassen, aus der Positionsinformation bezüglich des Markers 50 und einer bspw. mittels Steuerungseinheit 45 überwachten Position des mobilen Geräts 40 eine Bewegungsbahn bzw. Trajektorie zu ermitteln, die das mobile Gerät 40 zurücklegen muss, um die Start- oder die Stopp-Position einzunehmen.

Schritt S2 kann auch umfassen, mit den ersten Sensorsignalen SES ein Referenz-Koordinatensystem für das mobile Gerät 40 zu definieren. Bei dem Referenz-Koordinatensystem handelt es sich um ein drei-dimensionales, globales Koordinatensystem. Hierbei umfasst Schritt S1 das Erfassen von ersten Sensorsignalen SES von wenigstens zwei jeweils zwei-dimensionalen Markern 50 oder drei eindimensionalen Markern. Diese sind so angeordnet und ausgerichtet, dass sie gemeinsam die drei Achsen des Referenz-Koordinatensystems definieren.

Schritt S2 kann auch umfassen, aus den ersten Sensorsignalen SES umfassend Positionsinformation des Markers und einer bspw. mittels Steuerungseinheit überwachten Position des mobilen Geräts eine Bewegungsbahn bzw. Trajektorie zu berechnen, die das mobile Gerät 40 zurücklegen muss, bspw. um von einer Startposition in eine Stopp-Position zu gelangen. Mit anderen Worten umfasst Schritt S2, ein Steuersignal zu ermitteln, welches einen Bewegungspfad des mobilen Geräts 40 definiert. Der Bewegungspfad kann ein Bewegungspfad für eine mobile Komponente 70 des mobilen Geräts 40 sein, wobei sich auch Start- und Stopp-Position dann auf die mobile Komponente beziehen. Alternativ könne einer Vielzahl von Arbeitspositionen für die mobile Komponente ermittelt werden, welche die mobile Komponente im Verlauf eines medizinischen Workflows nacheinander einnehmen muss, welche zusammen betrachtet einen Bewegungspfad für die mobile Komponente definieren.

Schritt S2 kann auch umfassen, basierend auf den Sensorsignalen SES wenigstens einen Konfigurationsparameter für das mobile Gerät zu bestimmen, denn der Marker 50 ist so ausgebildet, dass er weitere Zusatzinformation für das mobile Gerät 40 codieren kann, um daraus für das mobile Gerät 40 mittels erster Sensorsignale SES Steuersignale STS für eine Konfiguration des mobilen Geräts ableiten zu können. Die Sensorsignale umfassen in dieser Ausführung einen, bevorzugt mehrere Konfigurationsparameter. Anhand des Konfigurationsparameters wird das mobile Gerät 40 (vor-)konfiguriert, voreingestellt bzw. in einen bestimmten Betriebsmodus versetzt. Der Konfigurationsparameter ist bspw. kennzeichnend für einen bestimmten Untersuchungs- oder Behandlungsraum, eine Untersuchungs- oder Behandlungsart oder für einen Nutzer sein, der eine Untersuchung oder Behandlung durchführt. Über die Codierung dieser Zusatzinformation in dem wenigstens einen Marker 50 entfällt eine manuelle, zeitintensive Konfiguration. Ein Benutzerabhängiger Betriebsmodus legt bspw. Benutzer-spezifische Geräteeinstellungen fest. Bspw. können die ersten Sensorsignale Identifikationsdaten eines Nutzers umfassen, wobei das auf diesen Identifikationsdaten basierende Steuersignal der präferierten Geräteeinstellung dieses Nutzers Rechnung trägt, bspw. kann derart die Stärke einer motorischen Bewegungsunterstützung bei teil-autonomen Bewegungen des mobilen Geräts 40 oder einer mobilen Komponente 70 automatisch voreingestellt werden. Dadurch steigt die Bedienfreundlichkeit.

Der Konfigurationsparameter kann auch ein Konfigurationsparameter bezüglich einer Netzwerkanbindung des mobilen Geräts sein. Dieser gibt bspw. an, welche Netzwerk-Anbindung in dem Untersuchungsraum für das mobile Gerät 40 zur Verfügung steht und/oder codiert Zugangs- bzw. Autorisierungsdaten für eine Verbindung des mobilen Geräts mit diesem Netzwerk. Über mittels dieses Konfigurationsparameters erstellte Steuersignale ist sichergestellt, dass sich das mobile Gerät 40 nach Erfassen der Sensorsignale des ersten Markers 50 direkt und automatisch für einen weiteren Datenaustausch in das Netzwerk einwählt. Bevorzugt erfolgt die Datenkommunikation über das Netzwerk kabellos.

Der Konfigurationsparameter kann zudem eine Konfiguration des mobilen Geräts bezüglich eines medizinischen Workflows festlegen. Der Workflow kann eine Kommunikation bzw. einen Datenaustausch mit anderen mobilen oder stationären Geräten in dem Untersuchungsraum erfordern. Die Sensorsignale SES kodieren hier Zusatzinformation zu dem medizinischen Workflow und die entsprechenden in Schritt S2 bestimmten Steuersignale bewirken automatisch den Aufbau einer Datenverbindung zwischen dem mobilen Gerät 40 und dem weiteren in den Workflow involvierten Gerät, insbesondere über das in dem Untersuchungsraum verfügbare, kabellose Netzwerk.

In Ausführungen, in denen das System 1 mehr als einen, also wenigstens einen zweiten Marker 51 umfasst, kann Schritt S1 auch das Erfassen von zweiten Sensorsignalen umfassen, wobei die zweiten Sensorsignale von dem zweiten Marker 51 erfasst werden. Schritt S2 umfasst dann das Bestimmen von zweiten bzw. weiteren Steuersignalen anhand der zweiten Sensorsignale. Insbesondere umfasst das Bestimmen der zweiten Steuersignale eine Korrektur bzw. eine Modifizierung der ersten Steuersignale. Bspw. kann anhand der zweiten Steuersignale eine zuvor bestimmte Position, Orientierung, Arbeitsposition und/oder ein zuvor bestimmter Arbeitspfad des mobilen Geräts 40 und/oder seiner mobilen Komponente 70 geändert/korrigiert werden. Dies ist insbesondere vorteilhaft, wenn der zweite Marker 51 an einem in der Umgebung des mobilen Geräts 40 befindlichen weiteren mobilen oder immobilen Gerät 80 angeordnet ist. Der zweite Marker 51 kann also insbesondere nach Detektion des ersten Markers 50 detektiert werden. Die Reihenfolge der Detektion ist jedoch durch die Erfindung nicht eingeschränkt und kann auch anders herum erfolgen.

In der Ausführung mit wenigstens zwei Markern 50 und 51 können die Schritte S1 und S2 wiederholt durchgeführt werden, wobei für jeden der Marker 50, 51 die Schrittfolge S1, S2 durchgeführt wird. In weiteren Ausführungen wird für jeden der Marker 50, 51 die Schrittfolge S1, S2, S3 durchgeführt, alternativ wird für beide Marker nur ein gemeinsamer Schritt S3 durchgeführt. Dies ist insbesondere von Vorteil, wenn schon während des Bestimmens von Steuersignalen basierend auf ersten Sensorsignalen (Schritt S2 des ersten Markers 50) der zweite Marker 51 detektiert wird bzw. zweite Sensorsingale SES erfasst werden (Schritt S1 des zweiten Markers). Insofern können einzelne Verfahrensschritte zumindest für unterschiedliche Marker gleichzeitig ablaufen und sich gegenseitig beeinflussen.

Der zweite Marker kann bspw. detektiert werden, wenn sich das mobile Gerät 40 entlang einer anhand erster Sensorsignale bestimmten Bewegungsbahn hin zu einer Start-Position bewegt. Der zweite Marker, insbesondere befindlich an einem weiteren mobilen Gerät, liefert nun zweite Sensorsignale, anhand derer anhand erster Sensorsignale bestimmte Steuersignale bezüglich der Bewegungsbahn des mobilen Geräts derart verändert und eine neue Bewegungsbahn ermittelt wird, damit das erste und das weitere mobile Gerät nicht kollidieren. In analoger Weise kann eine ursprüngliche Bewegungsbahn der mobilen Komponente 70 mittels zweiter erfasster Sensorsignale SES angepasst werden, um eine Kollision zu vermeiden.

Schritt S3 erfolgt in dieser Ausführung über die Kommunikations-Schnittstelle 46. Die Steuersignale STS werden insbesondere an das Fahrwerk 60 oder die mobile Komponente 70 ausgegeben.

In folgenden wird die vorliegende Erfindung nochmal kurz zusammengefasst:
Für automatisierte Arbeitsabläufe (engl. workflows) von mobilen, (teil-)autonomen Medizingeräten im Krankenhaus, ist es erforderlich, dass für derartige mobile Medizingeräte einfach Arbeitspositionen definiert werden können und diese Systeme sich anhand dieser Fixpositionen referenzieren können. Die Erfindung löst dieses technische Problem durch die Verwendung von flächigen Markern (QR-Codes, Barcodes etc.). Diese dienen vornehmlich der Definition von Referenzpositionen (und Ausrichtung) im Raum und zusätzlich der Hinterlegung von Zusatzinformationen in den Codes der verwendeten Marker. Die Erfindung besteht in der Integration von Kameras in Medizingeräte, welche Marker optisch erfassen können. Diese Marker können zusätzliche Informationen in Form von Codes (z.B. QR Code, Barcode, DataMatrix-Code o.ä.) enthalten, um einem mobilen, (teil-)-autonomen Medizingerät so Zusatzinformationen zur Verfügung stellen zu können. Ein Auswerteverfahren ermittelt aus den Kamerabildern der Marker
a) Ort und Orientierung der Marker für eine entsprechende Ausrichtung und Positionierung des mobilen, (teil-)autonomen Medizingerätes
b) ein globales Koordinatensystems sofern mindestens drei Marker erfasst wurden
c) Zusatzinformationen (Raum-, Workflow-, Statusinformationen) welche vom Medizingeräte für gerichtete Aktionen verarbeiten werden können.
Neben einer kamerabasierten Erfassung der Marker ist ferner eine optische (z.B. Laserscanner mit entspr. Markern), elektromagnetische (z.B. Hallsensoren mit entspr. Markern) oder taktile Erfassung (kapazitiv, induktiv, resistiv mit entsprechenden Sensoren) möglich.

Die Vorteile der Erfindung liegen im Wesentlichen in
- einer einfachen und zuverlässigen Lokalisierung (Ermittlung eines Koordinatensystems / einer Weltkarte) für mobile, (teil-)autonome Medizingeräte,
- einer einfachen und zuverlässigen Definition von Arbeitspositionen und Scanbahnen (z.B. Scanpositionen, Start-/Stopppositionen und Scanrichtung) für mobile, (teil-)autonome Medizingeräte durch den Menschen (sichtbar und nachvollziehbar),
- einer einfachen Datenspeicherung und -übertragung an mobile, (teil-)autonome Medizingeräte, um so Zusatzinformationen über die Umgebung oder Workflow bereitzustellen, um so Assistenzfunktionen oder automatisierte Vorgänge zu ermöglichen.

## Patentansprüche

1. Verfahren zur Steuerung eines mobilen medizinischen Geräts (40) umfassend
- eine Sensoreinheit mit zumindest einem Sensor (47) und
- eine Steuerungseinheit (42),
wobei das Verfahren folgende Schritte umfasst:
- Erfassen mittels des zumindest einen Sensors (47) von ersten Sensorsignalen (SES) betreffend wenigstens einen in einer Umgebung des mobilen medizinischen Geräts befindlichen Markers (50), und
- Bestimmen mittels der Steuerungseinheit (45) wenigstens eines Steuersignals (STS) für das mobile, medizinische Gerät basierend auf den erfassten ersten Sensorsignalen.

2. Verfahren nach Anspruch 1, wobei das Bestimmen des Steuersignals umfasst, basierend auf den ersten Sensorsignalen wenigstens eine Position und/oder eine Orientierung für das mobile Gerät relativ zu der Position des Markers zu bestimmen.

3. Verfahren nach Anspruch 2, wobei das Bestimmen des Steuersignals umfasst, basierend auf den ersten Sensorsignalen wenigstens ein Referenz-Koordinatensystem für das mobile Gerät zu definieren.

4. Verfahren nach einem der Ansprüche 2 oder 3, wobei das Bestimmen des Steuersignals ferner umfasst, basierend auf den ersten Sensorsignalen eine Arbeitsposition und/oder einen Bewegungspfad des mobilen Geräts zu ermitteln.

5. Verfahren nach Anspruch 4, wobei eine zuvor bestimmte Position, Orientierung, Arbeitsposition und/oder ein zuvor bestimmter Arbeitspfad basierend auf erfassten zweiten Sensorsignalen (SES) betreffend wenigstens einen in der Umgebung des mobilen Geräts befindlichen zweiten Marker (51) verändert wird.

6. Verfahren nach einem der vorhergehenden Ansprüche, wobei das Bestimmen des Steuersignals (STS) umfasst, basierend auf den Sensorsignalen wenigstens einen Konfigurationsparameter (KP) für das mobile Gerät zu bestimmen.

7. Verfahren nach Anspruch 6, wobei der Konfigurationsparameter eine Konfiguration des mobilen Geräts bezüglich einer Netzwerkanbindung festlegt.

8. Verfahren nach Anspruch 6 oder 7, wobei der Konfigurationsparameter eine Konfiguration des mobilen Geräts bezüglich eines Nutzers festlegt.

9. Verfahren nach einem der Ansprüche 6 bis 8, wobei der Konfigurationsparameter eine Konfiguration des mobilen Geräts bezüglich eines medizinischen Workflows festlegt.

10. Mobiles medizinisches Gerät (40) umfassend
- eine Sensoreinheit mit zumindest einem Sensor (47) zum Erfassen wenigstens eines ersten Sensorsignals, und
- eine Steuerungseinheit (45) zur Steuerung des mobilen medizinischen Geräts, wobei die Steuerungseinheit umfasst:
- eine Schnittstelle (46) zum Empfangen von mittels zumindest eines Sensors (47) erfassten ersten Sensorsignalen (SES) betreffend wenigstens einen in einer Umgebung des mobilen medizinischen Geräts befindlichen Markers (50),
- eine Bestimmungseinheit (49) zum Bestimmen wenigstens eines Steuersignals (STS) für das mobile, medizinische Gerät basierend auf den erfassten ersten Sensorsignalen, und
- eine Schnittstelle (46) zum Ausgeben des wenigstens einen Steuersignals an das mobile Gerät.

11. Mobiles medizinisches Gerät nach Anspruch 10, umfassend ein Fahrwerk (60) und/oder eine mobile Komponente (70), wobei die Steuerungseinheit ausgebildet ist, das Fahrwerk und/oder die mobile Komponente des mobilen Geräts zu steuern.

12. System umfassend
- ein mobiles medizinisches Gerät nach einem der Ansprüche 10 oder 11, und
- wenigstens ein en in einer Umgebung des mobilen medizinischen Geräts befindlichen Marker (50, 51).

13. System nach Anspruch 12, wobei der wenigstens eine Marker ein QR-Marker ist.

14. System nach Anspruch 12 oder 13, wobei der wenigstens eine Marker in einem Boden, an einer Wand oder einer Decke eines Untersuchungsraumes einer medizinischen Einrichtung angeordnet ist.
